# EUROPEAN PATENT SPECIFICATION

(11) **EP 1 830 668 B1**
(45) Date of publication and mention of the grant of the patent: **12.01.2011**
(21) Application number: 05820880.2
(22) Date of filing: 29.11.2005
(51) Int. Cl.: A23P 1/04, A23D 9/02, A23P 1/12, A23L 1/00

(54) **PROCESS FOR THE ENCAPSULATION OF POLYUNSATURATED FATTY ACIDS**
VERFAHREN ZUR VERKAPSELLUNG MEHRFACH UNGESÄTTIGTE FETTSÄUREN
PROCÉDÉ D'ENCAPSULATION D'ACIDES GRAS POLYUNSATURÉS

(30) Priority: 21.12.2004 EP 04106763; 13.04.2005 EP 05102892
(43) Date of publication of application: 12.09.2007
(73) Proprietor: Firmenich SA, 1211 Geneva 8 (CH)
(72) Inventor: VALENTINOTTI, Sergio, 1006 Lausanne (CH); ARMANET, Luc, LAWRENCEVILLE, NJ, 08648 (US); PORRET, Joëlle, 2224 Chêne-Bougeries (CH)
(74) Representative: Dale, Gavin Christopher
(86) International application number: PCT/IB2005/053939
(87) International publication number: WO 2006/067647

(56) References cited:
- WO-A-03/088755
- US-A- 4 232 047
- US-A- 4 707 367
- US-A- 5 972 395
- US-A1- 2004 017 017
- US-A1- 2004 121 154
- US-A1- 2004 234 673

## Description

### Technical Field

The present invention relates to a process for the preparation of particles comprising an oil rich in polyunsaturated fatty acids (PUFA), such particle, comprising an oil rich in PUFAs dispersed in a carbohydrate material.

### Background of the Invention and Problem to be Solved

The beneficial effects of polyunsaturated fatty acids (PUFAs) on human health have been confirmed repeatedly. Amongst the PUMAs, especially long-chain omega-3 fatty acids such as eicosapentanoic acid (EPA) or docosahexaenoic acid (DHA), for example, were shown to keep serum cholesterol levels low, stabilise irregular heart beat, reduce blood pressure, improve autoimmune diseases, improve depression disorders, and to prevent cancer of the colon.

Given these and other health benefits, it becomes a general interest to provide PUMAs as a functional additive to orally ingestible matter, such as food, nutritional supplements, beverages, pills, for example.

The addition of PUFAs to elements of human diet or supplements, however, is problematic due to the susceptibility of PUFAs to oxidation. In the presence of the ubiquitous oxygen, oils comprising PUFAs become quickly rancid and develop repellent odours and tastes, thus posing a hindrance to carefree consumption even in non-oxidised state.

At elevated temperatures the oxidation of PUFAs is even accelerated for reaction-kinetic reasons, which explains the difficulty of using PUFAs in food manufacturing or encapsulating processes that involve heat treatments.

Basic solutions to address the above problems propose the addition of antioxidants to oils rich in PUFAs. Daeseok et al, "Solubilisation of Vitamin C in Fish Oil and Synergistic Effect with Vitamin E in retarding Oxidation", JOACS, Vol. 68, No. 10, (October 1991) found synergistic effect of combined antioxidants vitamin E and C at ambient temperatures. Many prior art solutions for providing storable and stable preparations of PUFAs have, therefore, exploited the synergistic interaction of vitamin C and vitamin E in oils containing PUFAs.

Ock-Sook et al "Synergistic Antioxidative Effects of Tocopherol and Ascorbic Acid in Fish oil/Lecithin/Water system" conclude that at least 0.01-0.02% added ascorbic acid is required to obtain a considerable synergistic effect with added δ-tocopherol in stabilizing fishoil. These references, however, are silent on the behaviour of PUFAs at temperatures above 80°C, and, in addition, they are basically theoretical and not related to practical and more complex systems involving processing PUFAs in manufacturing processes for providing a medium- or long-term storable form of PUFAs.

In combination to the addition of antioxidants, oils rich in PUFAs have been encapsulated with the intention to prevent contact with oxygen and to provide a material that can easily be processed and combined with foods or other consumable matter. Preferred encapsulation systems include spray drying.

US 2003/00444490 A1 discloses a dry, stable oil composition comprising PUFAs, starch hydrolysate, converted starch and further, optional components, obtained by freeze- or spray drying emulsions based on the ingredients just mentioned. The process disclosed in this prior art document, however, seems to be very energy consuming due to the drying of emulsions having up to 60% water contents. In addition, this reference does not address the problem of oil remaining on the surface of the obtained spray dried particles, still prone to oxidation. Furthermore, spray-dried particles are often very small and porous and oxygen can quickly diffuse through them and get in contact with the PUFAs.

US 6,048,557 discloses water-soluble, porous carrier particles onto which PUFAs have been coated or absorbed. Coating or absorption of porous carriers, however, results in unprotected PUFAs on the surface of the particles, which exposes the PUFAs of this teaching to oxygen and thus makes this PUFA-preparation unsuitable for storage at ambient temperature.

A method for fixing a labile material in an extruded, glassy, moisture-stable substrate is taught in US 5,972, 395. Accordingly, a homogeneous substrate without any added moisture consisting of carbohydrates, sugar alcohols, and other ingredients in specified amounts is processed in a screw-extruder. However, screw-extruders operate at high pressures and under shear forces of the screws, which is generally detrimental to sensitive PUFAs. It would thus be advantageous to have a process avoiding the use of high pressures and shear forces in the preparation of capsules rich in PUFAs. In addition, the examples of this reference it emerges, however, that only low amounts (11% and less) of labile material could be encapsulated, if the labile material is not miscible in the other components. In view of this prior art it is desirable to provide a stable, powdered preparation comprising higher loads of PUFAs.

US-A1-2004/0234673 discloses a process of encapsulation in which an emulsion comprising a sensitive agent to be encapsulated is added to a powdered mixture of carbohydrate agents whereas US-A1-2004/0121154 relates to pellets in which an oily oxidation-sensitive active component is encapsulated in a matrix which comprises at least one water-soluble polysaccharide as film-forming agent. Neither document discloses preparation of a concentrated syrup prior to addition of an oil rich in polyunsaturated fatty acids.

WO-A1-03/088755 describes delivery systems for functional ingredients in which a blend is prepared comprising one or more carbohydrate, one or more hydrocolloid, one or more sugar, sugar alcohol or sugar syrup, or a mixture thereof, heating the blend, adjusting its moisture content, adding a functional ingredient to form a matrix and moulding the matrix. This document makes no reference to a cooling step and washing step.

In view of the prior art there is a need for providing PUFAs in a form that warrants stability of the PUFAs over a time range of several months at room temperature. In other words, the PUFAs should be encapsulated in way to allow their application to shelf-stable products. More particularly, there is a need to provide encapsulated PUFAs, wherein capsules provide a significant barrier to oxygen and have higher loads of oil rich in PUFAs to be encapsulated than comparable systems of the prior art. On the other hand, it is an objective to provide a possibility of encapsulating PUFAs in methods entailing high-temperature exposure to the PUFAs, preventing oxidation during the encapsulation process and the consequent development of off-tastes. In view of the many propositions for encapsulating PUFAs of the prior art, it is a further objective to provide a different method for encapsulating PUFAs, preferably being more cost efficient.

Furthermore, it is an objective to provide capsules having a sufficiently high glass transition temperature (T_{G}) to warrant stability at room temperature. Advantageously, T_{G} of a powder comprising encapsulating particles should be above 25°C, or even above 30°C.

Moreover, it is an objective to provide a process in which the above advantages are maintained on a pilot plant and/or industrial scale.

Furthermore, it is an objective of the invention to provide food products with PUFAs without modifying the organoleptic properties of the food product over storage time and/or shelf life.

### Summary of the Invention

Remarkably, the inventors of the present invention found a way to encapsulate an oil rich in PUFAs in a process entailing temperatures above 70°C and, if desired, even above 100°C. Surprisingly, the oil encapsulated by this process remains shelf stable over several months without developing malodours or off-tastes. The method of the invention provides advantageous capsules having relatively high loads of encapsulated oil but negligible amounts of residual oil on the surface of the capsules. In addition, the capsules surprisingly provide an efficient oxygen barrier and make them suitable to encapsulate oxidation-susceptible material.

Accordingly, the present invention provides a process for the preparation of a particles comprising an oil comprising polyunsaturated fatty acids (PUFA), the method comprising the steps of:
- adding water to at least one carbohydrate material to obtain an aqueous mixture;
- heating the aqueous mixture to form a concentrated syrup;
- emulsifying the oil rich in PUMAs, optionally comprising antioxidants, in the concentrated syrup to obtain an emulsion;
- extruding the emulsion through a die to obtain an extruded emulsion;
- cooling the extruded emulsion by putting or dropping it into a cold liquid to form a solid extruded material;
- washing the solid extruded material with a solvent liquid, and,
- drying it.

The particles produced by the process of the invention have the advantage to develop less or no fish-taste during a prolonged shelf-life, due to the very limited amount of surface oil. At the same time, it shows that the capsules of the present invention provide an effective barrier against oxygen, unlike other particles such as spray dried or screw-extruded ones.

### Description

Within the context of this specification the word "comprises" is taken to mean "includes, among other things". It is not intended to be construed as "consists only of".

In the context of the present invention, percentages are percentages by weight of dry matter, unless otherwise indicated. Similarly, if proportions are indicated as parts, parts of weight of dry matter are meant.

The term "oil rich in PUFAs" refers to an oil comprising at least 5wt.-% of PUFAs. Preferably, it is an oil comprising at least 10wt.-%, preferably at least 25wt.-% of PUFAs. For example, it is an oil comprising DHA and/or EPA.

Some of the basic process steps of the present invention have been reported from the prior art, without showing their suitability for encapsulation of oils rich in PUFAs. For example, US 4,707,367 illustrates a process for encapsulating an essential oil flavour composition including the steps of preparing an aqueous mixture, preparing an emulsion and extruding the same into a cold solvent. Therefore, this patent is explicitly incorporated herein by reference.

The process of the present invention comprises the step of adding water to at least one carbohydrate material to obtain an aqueous mixture. On the other hand, the particle of the present invention comprises an oil rich in PUFAs dispersed in a carbohydrate material.

As the carbohydrate material in the process and the particle of the present invention, any carbohydrate or carbohydrate derivative, which can be processed through extrusion techniques to form a dry extruded solid can be used.

Preferably, the carbohydrate material comprises at least one water-soluble carbohydrate. The term "water-soluble carbohydrate" means that the carbohydrate is at least 50% soluble according to the method described by L. Prosky et al., J. Assoc. Off. Anal. Chem. 71, 1017-1023 (1988).

Particular examples of suitable materials include those selected from the group consisting of sucrose, glucose, lactose, levulose, fructose, maltose, ribose, dextrose, isomalt, sorbitol, mannitol, xylitol, lactitol, maltitol, pentatol, arabinose, pentose, xylose, galactose, hydrogenated starch hydrolysates, maltodextrin, agar, carrageenan, other gums, polydextrose, cyclodextrin, synthetic polymers such as polyvinyl alcohol, semi-synthetic polymers such as succinylated starch, for example, alkenyl succinylated starch, cellulose ethers, and derivatives and mixtures thereof

Preferably, maltodextrin or mixtures of maltodextrin and at least one material selected from the group consisting of sucrose, glucose, lactose, levulose, maltose, dextrose, maltotriose, fructose, isomalt, sorbitol, mannitol, xylitol, lactitol, maltitol and hydrogenated starch hydrolysates will be used. Preferably, the maltodextrin has a dextrose equivalent (DE) of ≤20) and more preferably a DE of about 18.

Preferably, the carbohydrate material comprises from 30 to 70%, more preferably from 40 to 60% of maltodextrin.

In an embodiment of the present invention, the carbohydrate material comprises from 30 to 70%, more preferably from 40 to 60% by weight of carbohydrates having a molecular weight of >950.

Preferably, the carbohydrate material comprises from 40 to 60%, more preferably from 30 to 49% of sucrose.

In an embodiment of the present invention, the carbohydrate material of the particle of the invention, or used in the process of the present invention, comprises from 30% to 49% of carbohydrates having a molecular weight of < 950.

The above mentioned carbohydrate materials are hereby given by way of example and they are not to be interpreted as limiting the invention. Although different carbohydrates are mentioned above as specific examples, it is clear that any material which is extrudable and currently used as a matrix material in the production of extruded solids appropriate for applications comprising oils rich in PUFAs is adequate for the aim of the invention and is therefore hereby included in the latter.

Water is added to the carbohydrate material to obtain an aqueous mixture. Preferably, the aqueous mixture comprises about 12-40%, more preferably 18-30% of water. Taking into account that carbohydrate materials are generally hygroscopic and have residual water of about 2-4%, the actually added water may be less than the above indicate values.

The step of obtaining an aqueous mixture may be performed in a pressure resistant stirred vessel, to which both, the carbohydrate material and the water have been added.

The present invention further comprises the step of heating the aqueous mixture to form a concentrated syrup. Preferably, the aqueous mixture is heated sufficiently to allow water to evaporate from it. For example it may be heated to a temperature in the range of 110-135°C in a pressure resistant stirred vessel.

Water may be evaporated from the aqueous mixture until a concentrated syrup having from 3 -15%, preferably 4 -12% water is obtained.

The present invention provides particles comprising oils rich in PUFAs. Furthermore, the process of the present invention includes a step of emulsifying an oil rich in PUFAs in the concentrated syrup to obtain an emulsion.

Oils rich in PUMAs are commercially obtainable. Such oils may be of different origins such as fish or algae. It is also possible that these oils are enriched in the PUFA content via different methods such as molecular distillation, a process through which the concentration of selected fatty acids may be increased.

In an embodiment of the process or the particles according to the present invention, the oil rich in PUMAs comprises PUFAs selected from the group consisting of eicosapentaenoic acid (EPA), docosahexaenoic acid (DHA), Arachidonic acid (ARA), and a mixture of at least two of them.

The oil rich in PUFA may, optionally, be supplemented with an antioxidant. For example, the antioxidant-supplemented oil may comprise added ascorbic acid (vitamin C), tocopherol (vitamin E), or both of them. Tocopherol may be α-, γ-, or δ-tocopherol, or mixtures including two or more of these, and is commercially available.

Tocopherols are soluble in oils and may be easily added at amounts in the range of 0.05-2%, preferably 0.1-0.9%, of the supplemented oil comprising the antioxidant.

Ascorbic acid may be added at amount of 0.05-5% of the supplemented oil, for example.

Ascorbic acid is not readily soluble in oils, but may be solubilised therein, for example via reversed micelles using lecithin or phosphatidyl choline as a surfactant and water. See Han and Shin, "Antioxidative effect of Ascorbic Acid olubilized in Oils via Reversed Micelles", Journal of Food Science, Vol 55, No. 1, 1990, 247-249.

In an embodiment of the present invention, the oil comprising PUFAs contains less than 1% by weight of added ascorbic acid. Preferably, the oil comprises less than 0.5% of added ascorbic acid. More preferably, the oil comprises less than 0.05% of added ascorbic acid. Most preferably, the oil is free of ascorbic acid.

Surprisingly, the inventors of the present invention found that the stability of the oil comprising PUFAs could be maintained, even at elevated temperatures, although no or only very little ascorbic acid was added. Equally, the stability of the oil comprising PUFAs could be maintained, although no or only little tocopherol (0.1 - 0.9 wt.-% of oil) was added. Without wishing to be bound by theory, the present inventors believe that by adding sufficient amounts of lecithin, no or only little amounts of antioxidants needs to be added to protect the oil rich in PUFAs from oxidation. It was hypothesised that lecithin, if added in sufficient amounts, could work itself as an antioxidant and/or sufficiently enhance the antioxidant properties of residual tocopherol, that is, tocopherol naturally present in the oil rich in PUFAs or added in amounts of 0.1 - 0.9 wt.%. This is an important advantage, also due to the fact that lecithin is more easily available than ascorbic acid or tocopherol and thus much less expensive.

Therefore, the present invention relates, in an aspect, to a method for preventing oxidation and/or for increasing stability of PUFAs in oils at temperatures above 70°C, more preferably above 90°, above 100°C, above 110°C, even above 120°C and up to 135°C, the method comprising the step of adding to the oil at least 1.5% of lecithin by weight of the oil rich in PUFAs prior to exposure of the oil to the temperatures given above. Preferably, at least 2% by weight of lecithin is added, and more preferably even more, as indicated by the ranges given below.

Therefore, in an embodiment of the process of the present invention, the oil rich in PUFAs further comprises 1.5-15% of added lecithin. Preferably, the oil comprises 3-12%, more preferably 4-10% of added lecithin, by weight of the oil rich in PUFAs optionally supplemented with ascorbic acid and/or tocopherol.

Preferably, the oil rich in PUFAs is mixed with the concentrated syrup under relatively low shear forces to uniformly disperse the oil rich in PUFAs throughout the concentrated syrup. For example a stirred vessel may be used to perform this step. However, any other way of preparing the emulsion may be suitable for example preparing a microemulsion with the oil rich in PUFAs and mixing it into the concentrated syrup.

Therefore, in an embodiment of the particle of the present invention, the oil rich in PUFAs, during the preparation of the particle, has been exposed to temperatures above 100°C, more preferably above 110°C, and even more preferably above 120°C.

The extruding step comprises the forcing of the emulsion through the holes of the die, thus forming strands of the extruded emulsion, which may later fall into a cold liquid, for example a cold solvent bath. Extrusion may be forced using gas or mechanical pressure. The extrusion pressure is preferably in the range of from 1.5 to 7x10⁵ Pa, preferably 1.5 to 3x10⁵ Pa. The force for extruding may be supplied by a pump, for example a gear-pump operating at a fixed speed resulting in a constant extrusion rate, or by pressurized air or a gas, such as pressurized nitrogen, for example.

The holes in the die plate may have a diameter adjusted to the final application of the capsules of the present invention. Preferably, the holes have a diameter of 0.3 - 5mm, more preferably, 0.5 - 2mm, for example.

In an embodiment of the process of the present invention, the emulsion, when leaving the die and before being cooled in the cool liquid, is **characterised in that** it has a temperature of 100 to 135°C, preferably 110 to 130°C, more preferably 115-130° and most preferably 120-130°C. Actually, this temperature was already obtained within the extrusion vessel and expresses the fact that the oil rich in PUFAs within the emulsion is exposed to temperatures above 100°C and up to 130°C, or even up to 135°C.

In a further step, the present invention provides the cooling by putting or dropping it into a cold liquid to form a solid extruded material. Preferably, the extruding step results in vertically extruded strands that are guided by gravity to a beneath-placed bath of a cold liquid. The cold liquid is preferably present within a vessel suitable to withhold liquids in the range of -200 to 100°C. The vessel may contain a blade impeller allowing the stirring of the cold liquid, and, at the same time, the disintegration of the strands of the extruded emulsion reaching the cold liquid. In the cold liquid, the strands are thus chilled and broken into smaller particles.

The cold liquid may be a cold organic solvent, such as hexane, for example. Preferably, the organic solvent is isopropanol. Alternatively, the cold liquid may be liquid nitrogen. Alternatively, the cold liquid may be limonene, and/or a plant extract of citrus-fruits comprising high amounts of limonene. The cold liquid is preferably held in a stirred vessel. In addition, the cold liquid may me a mixture of several solvents. Preferably, the cold liquid comprises limonene and isopropanol. More preferably, the cold liquid comprises 5-30% isopropanol and 95-70% limonene.

Preferably, the cold liquid has a temperature in the range of 20 to -200°C, more preferably 15 to -80°C, most preferably 5 to - 20°C. Generally, the temperature is sufficiently low as to permit the forming of a solid, glassy state from the extruded emulsion. Preferably, the temperature is below the boiling point of the cold liquid. This temperature preferably is the temperature of the cold liquid before the extruded emulsion is dropped into it.

During cooling, the particles are forming a solid extruded material. In particular, the cooling occurs so quick that the extruded emulsion immediately transforms into a solid, glassy state.

Thereafter, the particles are removed from the cold liquid, for example by centrifugation or sieving. Therefore, in an embodiment, the process of the present invention comprises a step of separating the solid extruded material from the cold liquid. This step may be easily carried out by providing an outlet valve and a sieve located upstream the outlet valve within the vessel containing the cold liquid. In this case, after having sufficiently cooled, the solid extruded material may be separated from the cold liquid by simply emptying the vessel by letting the cold liquid leave through the outlet valve.

In a further step, the present invention provides washing the solid extruded material. Preferably, the solid extruded material, which preferably has the form of particles, is washed in a solvent liquid. Preferably, the solvent liquid is suitable to substantially remove surface oil, located on the surface of the particles formed by the glassy material.

Preferably, the solvent liquid is an extract from citrus fruits rich in limonene. Limonene is present in the rind of citrus fruits. During production of juices, the oil of the rind is separated, and, valuable flavours are fragranced are recovered. The bulk of the oil, however, is limonene, which traditionally is disposed off. Surprisingly, this abundantly and inexpensively available waste product of citrus-oil production is particularly suitable to remove surface oil from the solid extruded material. In this way, the present inventors found a very useful and advantageous use for limonene. Therefore, in an embodiment of the process according to present invention the solvent liquid comprises terpenes obtained from citrus fruits.

Therefore, the particles that have been separated from the cold liquid above may be transported into vessel containing a solvent, preferably limonene. The washing takes preferably place under agitation or stirring in a way that the particles are not further disintegrated or broken apart, for example, by slowly stirring the solvent with a blade impeller not getting into contact with the particles. After the washing step, the solvent liquid may be removed as described above for the cold liquid.

In an embodiment of the process of the present invention, the cooling step and the washing step are both performed in the same cold solvent liquid. The inventors of the present invention have thus surprisingly found that during the step of cooling the extruded material to form a solid glassy material, the surface oil can be effectively removed at the same time.

Even more surprisingly, the cooling and the washing step can both be conducted in solvents comprising plant extracts rich in limonene mentioned before. This has the particular advantage that only a natural cold solvent liquid is used, which does not have to be totally removed subsequently and which is unproblematic from a handling and regulatory point of view. In addition, the use of natural plant extracts rich in limonene in the process of the present invention is far less expensive than the use of traditional solvents. Preferably, the limonene, or the solvent comprising limonene has a temperature as the cold liquid defined above.

However, the cooling and the washing step may be performed within the same process step in a cold solvent liquid which may also be isopropanol, liquid nitrogen, hexane, others, or mixtures of two or more of these, for example. For example, the cold solvent liquid may be free of limonene.

This embodiment, where the cooling and washing step are performed in the same cold solvent liquid has a substantial advantage over prior art process for encapsulating oils rich in PUFAs, for example by prior art screw-extrusion processes, because the use of a cold solvent directly after extrusion first results in a denser matrix, resulting in a more effective oxygen barrier and thus increased stability, while at the same time (and in the same step) effectively removing surface oil and thus preventing off-flavours based on oxidised surface oil.

In a further step, the process of the present invention comprises drying the washed, solid extruded material. This step may be performed to remove residual solvent from the particles. Suitable drying apparatuses could be multiple tray type dryers, rotary drum driers or fluidised bed dryers, for example, with typical residence times of 1-8 hours (rotary drum) or 30 - 60 minutes (fluidised bed), respectively.

Preferably, particles of the washed solid extruded material are dried to achieve a water content in the range of 2 - 7% by weight of the capsules including the water.

During the drying step an anticaking agent may be added. Once the particles have been dried, they may further be mixed with a free-flowing agent and subsequently sifted to meet size specification.

The particles obtained by the process of the present invention are found to have a low surface content of residual oil, which is ≤ 0.2% of the total weight of the particles, preferably, the surface oil is ≤ 0.1%, more preferably ≤ 0.08, even more preferably ≤ 0.05, and most preferably ≤ 0.04% by weight of the total weight of the particles. The residual oil on the surface, also called surface oil hereinafter, is determined by the following protocol:

### 1) Solutions

For preparing a calibration curve: estimate the residual surface oil content, establish solutions of the oil rich in PUFAs in hexane including the expected content. If possible, use the same oil used in the encapsulation system. If not possible, use oil that has the same or very close properties.

### 2) Standards

5.0000g of particles are put into an Erlenmeyer.

15 mL of hexane are added and the Erlenmeyer is immediately stirred for 20 minutes (without magnetic bar to avoid breaking the particles).

The solvent-powder mix is filtered into a 25 mL measuring flask

The Erlenmeyer is washed with 2 x 5 mL of hexane, the filter is washed with 1 x 2 mL of hexane, the volume is adjusted to exactly 25 mL.

### 3) Injection conditions

HPLC MERCK
Diode array detector MERCK L-7450
Column thermostst L-5020
Autosampler AS-4000
Loop : 20 µl
Use the pump isocratically at 1,0 ml/min flow rate
Solvent B : hexane (50)
Solvent C : tetrahydrofuran (50)
Pressure limit:
   min : 2 bars
   max : 250 bars
Run : 5 min

### 4) Calculation

A calibration curve is determined based on the information obtained in point 1) above. The percentage of surface oil of the samples can be calculated based on the calibration curve.

The particles obtained by the process of the present invention are, surprisingly, found to provide an effective barrier to oxygen, which may be explained, without wanting to be bound by theory, by the relatively high density of the amorphous matrix built up by the carbohydrate material during the step of cooling the extruded emulsion in the cold liquid. The particles of the present invention differentiate from those obtained by screw extrusion, because in the latter usually higher pressures are used, resulting in greater expansion of the extruded emulsions immediately after the extrusion holes. In these processes, particles with lower densities of the carbohydrate glassy material are obtained, providing a less efficient oxygen barrier.

Therefore, in an embodiment, the particle obtained by the process of the present invention has a density of ≥ 1.3 g/cm³, preferably ≥ 1.35 g/cm³ based on an oil content of 10%. The density is determined based on the dry weight of the particles.

In an embodiment, the particle obtained by the process of the present invention is obtainable by the process of the present invention. More preferably, it is obtained by this process.

Preferably, the particles obtained by the process of the present invention have a glass transition temperature (T_{G}) above 20°C, more preferably above 25°C, even more preferably above 30°C and most preferably above 37°C. T_{G} was determined with a Perkin-Elmer DSC 7. Samples (about 10 mg each) were cooled to -20°C and held for 5 minutes. Temperature was ramped at 10°C/min to 120°C followed by quenching at -20°C. After a 5-minute hold, the temperature was ramped to 120°C at a rate of 10°C/minute. T_{G} was determined by the inflection of the heat flow curve of the rescan. Duplicate samples of each product were run.

The present invention relates to a food product comprising the particles obtained by the process of the invention. In an embodiment, the food product has a water activity of below 0.5. Preferably, the food product has a water activity below 0.45, below 0.4, below 0.35 or even below 0.3. Most preferably, the water activity is below 0.25, 0.2, 0.15 or even below 0.1. With relatively low availability of free water in a food product, as is the case with the parameter of water activity as set out above, the matrix of the particles of the invention remains intact for a longer time and thus better protects the oil rich in PUFAs from oxygen.

Water activity is preferably measured with an Aqualab CX-2 apparatus (Decagon Devices, Inc., Pullman, Washington, USA). The apparatus is to be used according to the user's manual. In particular the thermostatic water bath connected to the apparatus is adjusted to 20°C. Start the procedure once the sample has been made thermostatic in the chamber foreseen for this. At the end of the procedure, check that temperature still is at 20 ±0.5 °C.

In an embodiment, the food product is selected from the group consisting of an instant soup, a breakfast cereal, a powdered milk, a baby food, a powdered junior drink, a powdered chocolate drink, a spread, a powdered cereal drink, a chewing gum, an effervescent tablet, a cereal bar, and a chocolate bar.

The powdered milks or drinks are products, which are usually consumed after reconstitution of the product with water, milk and/or a juice, or another aqueous liquid. The baby food may be an infant formula, for example.

The food product preferably is a particulate or powdery food, and the particles of the invention may easily be added thereto by dry-mixing. Preferably, the particles are added in an amount, which provides 10-100%, preferably 20-80% of the recommended daily allowance (RDA) of PUFAs per serving size of the food product. More preferably, a serving of the food product provides the above percentages of RDA of DHA.

The following examples represent particular embodiments of the present invention without limiting its general scope.

### Example 1

### Preparation of Particles Comprising Fish-Oil

A 20wt.-% aqueous solution of gum arabic is prepared. 3.166kg of the solution is mixed with 3.66kg of water in a tank suitable to withstand pressures of up to 10 bars and having, on its bottom, an outlet valve with die holes. The tank is equipped with a mechanical stirrer.

To this solution, 7.5 kg of maltodextrin with DE = 18, 9.96 kg of sucrose and 16 g of lecithin (=10% of total lecithin) are added.

The resulting aqueous mixture of carbohydrates is heated under stirring until a concentrated syrup having about 8-10% water content is obtained. This occurs at about 115°C.

In parallel, 140 g of lecithin are dissolved in 1.7 kg of fish oil rich in polyunsaturated fatty acids. The resulting oil is emulsified in the concentrated syrup under stirring. Thus, an emulsion is obtained.

The emulsion is then heated to about 130°C and the tank is pressurized with nitrogen up to 5 bars. Thereafter, the outlet bottom valve is opened and the emulsion is thus pushed through the die and forms long thin strands, which are falling into a vessel equipped with a blade-impeller and containing isopropanol at -4°C.

The strands of the extruded emulsion, when dropping into the agitated cool isopropanol, solidify and are subsequently disintegrated into a glassy material having the form of small rods.

The Isopropyl alcohol is removed from the disintegration reactor through an outlet valve. The small rods are retained in the vessel thanks to a fine sieve located before the outlet valve.

One half of all of the glassy material is retained in the vessel and the other half is removed and dried as described further below.

Two parts of limonene are added to the vessel containing one part of rods. The agitation is again initiated in a way that the rods in the reactor are not further disintegrated. This process is referred to as washing process.

The washing process lasts for 10min. After this time the limonene is evacuated through the vessel outlet valve.

The small rods washed with limonene are placed in a drum drier and 1wt.-%g of an anti-caking agent (SiO₂) are added. Drying is performed at 80°C for 8 hours.

The rods that were recovered from the disintegrator reactor before the addition of limonene are also placed in a drum drier, supplied with an anti-caking agent and dried as described above.

Once the drying process is terminated a 10 grams sample of rods from each drum drier is taken to analyse the total oil content as well as the surface oil content. It was found that the glassy material in the form of rods comprised 10wt.-% of fish oil, in the washed as well as in the non-washed sample.

For assessing the surface oil, the method according to the description is used. Accordingly, the oil that remains on the surface of the glassy material in the non-washed rods is in the range of 0.1-0.5% of the total weight of the glassy material. The sample that has been further washed with limonene had a surface oil content in the range of 0.01-0.05% of the total weight of the glassy material.

### Example 2

Particles having a load of oil rich in PUFAs of 15%by weight of the total capsules are prepared. Accordingly, the ingredients in the table below were processed according to the protocol of Example 1.

| Ingredient | Weight in kg |
|---|---|
| Maltodextrin DE 18 | 7.07 |
| Sucrose | 6.53 |
| Lecithin | 0.26 |
| Oil rich in PUFAs | 2.55 |
| Gum arabic | 0.59 |

### Example 3

The particles obtained in Examples 1 and 2 were stored at 30°C for 6 months. At regular intervals, the particles were tested by sniffing. During the period of six months, no fish-odour or typical rancid smell could be noticed.

### Examples 4 - 34

### Food Product Comprising the Encapsulated Fish Oil

Commercially obtainable food products having a water activity below 0.5 were dry-mixed with varying quantities (2-5g) of the particles obtained in example 1. Effervescent tablets, compressed tablets were made following standard recipes and procedures devoid of active principles (only standard filler materials). Similarly, cereal bars were made with state of the art procedures and ingredients. Two (2) grams of these particles correspond to 30% of the Recommended Daily Intake of DHA.

The products were tested, after reconstitution or short cooking if applicable, for fishy taste by sniffing (10-30 persons). Table 1 below lists the commercially obtainable food products and the food-category to which they belong. In addition, the table lists the water activity of the respective food products, the amount of particles added per quantity of the respective food product and the summarized outcome of the sensory evaluation.

In all products having a water activity below 0.5, no fishy taste was observed upon consumption.

**Table 1: Food Products Comprising the Particles of the Present Invention**

| **Example** | **Commercially Obtained Food** | **Product Category** | **Amount of Particles** | **Comments** | **Aw** |
|---|---|---|---|---|---|
| 4 | Maggi ® soup Wellness 3 cér (4 x 20 g) | instant soup | 1g and 2 g per serving (20 g) | not fishy, citrus, lemon taste | 0.03 |
| 5 | Maggi ® soup Wellness 9 veg (4 x 20 g) | instant soup | 1g and 2 g per serving (20 g) | not noticeable (not fishy), citrus, lemon taste | 0.03 |
| 6 | Japanese noodlesoup shrimp (100 g) | instant soup | 2 g per 100 g (1 serving) | softer flavor, no taste of fish/good taste | 0.03 |
| 7 | Instant noodle prawn flavor soup (100 g) (Migros) | instant soup | 2 g per 100 g (1 serving) | not noticeable/good taste | 0.03 |
| 8 | Knorr® Spaghetteria All'Arrabbiata (201 g) | instant soup | 2 g per 100.5 g (1 serving) | not fishy | 0 |
| 9 | Uncle Ben's® express Mediterranean rice (250 g) | instant soup | 2 g per 125 g (1 serving) | not fishy | 0 |
| 10 | Uncle Ben's® express indian rice (250 g) | instant soup | 2 g per 125 g (1 serving) | not fishy | 0 |
| 11 | Maggi® sweet & sour mix (66 g) | instant sauce | 2 g per 22 g (1 serving) | not fishy | 0.03 |
| 12 | Maggi® mah meeh mix (29 g) | instant sauce | 2 g per 29 g (1 serving) | Very good, not much difference | 0.03 |
| 13 | Maggi® curry mix (40 g) | instant sauce | 2 g per 40 g (1 serving) | not fishy | 0.03 |
| 14 | Maggi® quick lunch funghi (61) | instant soup | 2 g per serving | creamier, more buttery, stronger mushroom taste | 0.03 |
| 15 | Knorr® sauce pour pasta al funghi (37 g) | instant sauce | 2 g per serving (2 servings) | not fishy, lemon taste, citrus | 0.03 |
| 16 | Knorr® quick soup asperges (49 g) | instant soup | 2 g per serving (3 x 1 portion) | citrus, sweeter | 0.03 |
| 17 | Knorr® quick soup orge (57 g) | instant soup | 2 g per serving (3 x 1 portion) | stronger taste of bacon, more salt than others | 0.03 |
| 18 | Knorr® quick soup veg (44 g) | instant soup | 3 g per serving (3 x 1 portion) | sweet taste, weaker, sweeter | 0.03 |
| 19 | Bossy® Swiss Muesli (1 kg) | cereal | 2 g per 100 g muesli (cold) /1 g per 50 g muesli (cold/boiling); 150 ml milk added | not fishy | 0 |
| 20 | Compressed tablets (made in-house, standard recipe) | | 2 g per 100 g tablets | not fishy | 0 |
| 21 | Quick® milk powdered milk (300 g) | Baby food, powder | 2 g per 20 g powdered milk diluted in 200 ml of water (or milk) 1 serving | not fishy | 0.03 |
| 22 | Nestle® Lactoplus Honey (300 g) | Baby food, powder | 2 g per 24 g per 250 ml of milk 45 °C | not fishy | 0.03 |
| 23 | Nestle® Junior Drink Vanille Cereals (375 g) | Baby food, powder | 2 g per 250 ml milk + 3 tablespoons of water 1 serving | not fishy | 0.03 |
| 24 | Nutella® 400 g (each slice of bread has 15 g of Nutella) | spread | 2 g per 30 g of Nutella | not fishy | 0.03 |
| 25 | Wander® Ceralino cereal drink with ovomaltine (350 g) | cereal | 2 g per 31 g diluted in 160 ml of milk and 80 ml of water 50°C | not fishy | 0.03 |
| 26 | Milupa Aptamil HA2® (2 x 300 g) | Baby food, powder | 2 g per 36.4 ml diluted in 210 ml of water 60°C | not fishy | 0.03 |
| 27 | Milupa® miluvid plus semoule lactée (275 g) | Baby food, powder | 2 g per 45 g diluted in 130 ml of water 50°C | not fishy | 0.03 |
| 28 | Nestle® Baby Menu céréales yogo-framboise (300 g) | Baby food, powder | 2 g per 60 g diluted in 150 ml of water 60°C | not fishy | 0.03 |
| 29 | Nestle® Baby Menu bouillie hypoallergénique (2 x 300 g) | Baby food, powder | 2 g per 65 g diluted in 130 ml of water 60°C | not fishy | 0.03 |
| 30 | Chewing gum triacitine 1% | | 3 gper 150 g gum | not fishy | <0.1 |
| 31 | Effervescent tablets (made in house, standard recipe) | | 4 g per 200 g tablets | not fishy | <0.1 |
| 32 | Cereal bars (made in house, standard recipe) | | 5 g per 250 g cereal | not fishy | 0.2 |
| 33 | Dark chocolate bars | | 5 g per 250 g choc | good, not fishy | 0 |
| 34 | Ovomaltine® (15 sachets à 15 g) | | 2 g per 250 ml of milk | not fishy | 0.03 |

## Claims

1. A process for the preparation of particles comprising an oil rich in polyunsaturated fatty acids (PUFA), the method comprising the steps of:
- adding water to at least one carbohydrate material to obtain an aqueous mixture;
- heating the aqueous mixture to form a concentrated syrup;
- emulsifying the oil rich in PUFAs, optionally comprising antioxidants, in the concentrated syrup to obtain an emulsion;
- extruding the emulsion through a die to obtain an extruded emulsion;
- cooling the extruded emulsion by putting or dropping it into a cold liquid to form a solid extruded material;
- washing the solid extruded material with a solvent liquid, and,
- drying it.

2. The process according to Claim 1, in which the cooling step and the washing step are both performed in the same cold solvent liquid.

3. The process according to Claim 1 in which the oil rich in PUFAs contains less than 1 wt.-% of added ascorbic acid.

4. The process according to Claim 1, in which the oil rich in PUFAs further comprises 1.5-15 wt.-% of lecithin.

5. The process according to Claim 1 in which the solvent liquid comprises terpenes obtained from citrus fruits.

6. The process according to Claim 1, in which the oil rich in PUFAs comprises PUFAs selected from the group consisting of eicosapentaenoic acid (EPA), docosahexaenoic acid (DHA), Arachidonic acid (ARA), and a mixture of at least two thereof.

## Patentansprüche

1. Verfahren für die Herstellung von Teilchen, umfassend ein Öl, reich an mehrfach ungesättigten Fettsäuren (PUFA), wobei das Verfahren die Schritte umfaßt:
- Hinzufügen von Wasser zu mindestens einem Kohlenhydratmaterial, um ein wässeriges Gemisch zu erhalten;
- Erhitzen des wässerigen Gemischs, um einen konzentrierten Sirup zu erzeugen;
- Emulgieren des an PUFAs reichen Öls, gegebenenfalls umfassend Antioxidantien, in dem konzentrierten Sirup, um eine Emulsion zu erhalten;
- Extrudieren der Emulsion durch eine Düse, um eine extrudierte Emulsion zu erhalten;
- Abkühlen der extrudierten Emulsion, indem sie in eine kalte Flüssigkeit eingegeben oder eingetropft wird, um ein festes extrudiertes Material zu erzeugen;
- Waschen des festen extrudierten Materials mit einer Lösungsmittelflüssigkeit und
- Trocknen.

2. Verfahren gemäß Anspruch 1, bei welchem der Schritt des Abkühlens und der Schritt des Waschens beide in der gleichen kalten Lösungsmittelflüssigkeit durchgeführt werden.

3. Verfahren gemäß Anspruch 1, bei welchem das an PUFAs reiche Öl weniger als 1 Gew.-% zugesetzte Ascorbinsäure enthält.

4. Verfahren gemäß Anspruch 1, bei welchem das an PUFAs reiche Öl weiterhin 1,5-15 Gew.-% Lecithin umfaßt.

5. Verfahren gemäß Anspruch 1, bei welchem die Lösungsmittelflüssigkeit aus Citrusfrüchten erhaltene Terpene umfaßt.

6. Verfahren gemäß Anspruch 1, bei welchem das an PUFAs reiche Öl PUFAs, ausgewählt aus der Gruppe, bestehend aus Eicosapentaensäure (EPA), Docosahexaensäure (DHA), Arachidonsäure (ARA) und einem Gemisch von mindestens zwei davon, umfaßt.

## Revendications

1. Procédé de préparation de particules comprenant une huile riche en acides gras polyinsaturés (AGPI), ledit procédé comprenant les étapes consistant à:
- ajouter de l'eau à au moins une matière glucidique pour obtenir un mélange aqueux;
- chauffer le mélange aqueux pour former un sirop concentré;
- émulsionner l'huile riche en AGPI, comprenant éventuellement des antioxydants, dans le sirop concentré pour obtenir une émulsion;
- extruder l'émulsion à travers une filière pour obtenir une émulsion extrudée;
- refroidir l'émulsion extrudée en la mettant ou en la faisant tomber dans un liquide froid pour former une matière extrudée solide;
- laver la matière extrudée solide avec un liquide solvant, et,
- sécher celle-ci.

2. Procédé selon la revendication 1, dans lequel l'étape de refroidissement et l'étape de lavage sont toutes deux effectuées dans le même liquide solvant froid.

3. Procédé selon la revendication 1, dans lequel l'huile riche en AGPI contient moins de 1% en poids d'acide ascorbique ajouté.

4. Procédé selon la revendication 1, dans lequel l'huile riche en AGPI comprend en outre 1,5-15% en poids de lécithine.

5. Procédé selon la revendication 1, dans lequel le liquide solvant comprend des terpènes obtenus à partir d'agrumes.

6. Procédé selon la revendication 1, dans lequel l'huile riche en AGPI comprend des AGPI choisis dans le groupe constitué de l'acide éicosapentaénoïque (AEP), de l'acide docosahexaénoïque (DHA), de l'acide arachidonique (ARA), et d'un mélange d'au moins deux d'entre eux.
